# EUROPEAN PATENT SPECIFICATION

(11) **EP 2 407 582 B1**
(45) Date of publication and mention of the grant of the patent: **07.06.2017**
(21) Application number: 10007363.4
(22) Date of filing: 15.07.2010
(51) Int. Cl.: C25F 3/16, A61L 31/02

(54) **Method and electrolytic solution for electropolishing stents made of high strength medical alloys**
Verfahren und Elektrolytlösung zum Elektropolieren von Stents aus hochfesten medizinischen Legierungen
Procédé et solution pour le polissage électrolytique de stents faits d'alliages médicaux à haute résistance

(43) Date of publication of application: 18.01.2012
(73) Proprietor: Abbott Laboratories Vascular Enterprises Limited, Dublin 2 (IE)
(72) Inventor: Wulf, Elena, 72393 Burladingen (DE)
(74) Representative: Peters, Hajo

(56) References cited:
- GB-A- 821 229
- GB-A- 1 357 978
- US-A1- 2007 209 947
- US-B1- 6 679 980

## Description

### BACKGROUND OF THE DISCLOSURE

The present disclosure relates generally to providing a method as well as an electrolytic solution for electropolishing products made from metals, and in particular, electropolishing metallic medical devices such as stents, made of high strength medical alloys like alloys including e.g. titanium, tungsten, tantalum, niobium, cobalt-chromium, etc. While the method and electrolytic solution are described herein as being applicable mainly to medical stents, in particular intravascular stents, the disclosure is not limited to such medical products. For example, the methods may be applied to electropolish metallic automotive or aerospace components.

Stents are generally tube-shaped devices placed within a blood vessel or other body lumen to maintain the patency of the lumen and, in some cases, to reduce the development of restenosis. The stents may be formed in a variety of configurations which are typically expandable since they are delivered in a compressed form to the desired site. Such a configuration may be a helically wound wire, wire mesh, weaved wire, serpentine stent, chain of rings, other configuration, or combinations thereof. The walls of stents are typically perforated in a framework design of wire-like connected elements or struts or in a weave design of cross-threaded wire. Some stents are made of more than one material. The stent may be, for example, a sandwich of metals having outer layers of a biocompatible material, such as cobalt chromium, with an inner layer providing the radiopacity to the stent for tracking by imaging devices during placement. A stent made of such material may be, for example, a thin layer of titanium between layers of cobalt chromium. In forming such stents from metal, a roughened outer surface of the stent may result from the manufacturing process. It may be desirable for the surface of the stent to be smooth so that it may be more inserted and traversed with reduced friction through the blood vessels or other body lumens toward the site of implantation. A rough outer surface may not only increase frictional obstruction, but may also damage the lining of the vessel wall during insertion. Furthermore, smooth surfaces may reduce thrombus formation and/or corrosion.

Since processing to form metallic stents often results in a product initially having burrs, sharp ends, debris, slag material from melting the metal during processing, other features, or combinations thereof, as a first order treatment of the product, the surface may be descaled in preparation for, for example, further surface treatment such as electropolishing.

A method or electrolytic solution is provided for electropolishing stents after they have been descaled, for example, by the method disclosed in US 2007209947 A.

Descaling may include, for example, dipping the stent into a strongly acidic solution and/or ultrasonically cleaning the stent.

Electropolishing is an electrochemical process by which some of the surface metal may be electrolytically dissolved. In general, the metal stent serves as an anode and is connected to a power supply while immersed in an electrolytic solution having a metal cathode connected to the negative terminal of the power supply. Current therefore flows from the stent, as the anode, causing it to become polarized. The rate at which the metal ions on the stent are dissolved may be controlled by the applied current and/or voltage. The positioning of the cathode relative to the stent may provide an even distribution of current to the stent. According to the theory of electropolishing, the current density is typically highest at high points protruding from a surface and is typically lowest at the surface low points. Thus, the higher current density at the raised points may cause the metal to dissolve faster at these points, which may level the surface. Electropolishing therefore may smooth the surface, even to the point where it is shiny and reflective.

The present disclosure provides a method and use of an electrolytic solution for electropolishing a plurality of metallic devices such as stents simultaneously to consistently produce smooth surfaces.

### BRIEF SUMMARY

An embodiment of a method is provided for electropolishing a metallic stent, preferably for simultaneously electropolishing a plurality of metallic stents. The method may include:
a) affixing a stent on each of one or more electrically conductive adaptors of an apparatus, the apparatus including:
   an elongated member having a longitudinal axis, preferably a plurality of elongated members having a longitudinal axis, the or each of the members including an electrically conductive adaptor capable of being removably affixed to and in electrical contact with a metallic stent;
   an electrolytic solution comprising dimethylsulfate;
   a continuous cathode configured to be located in close proximity to the or each of the elongated members when the elongated member/s and cathode are immersed in the electrolytic solution;
   a cathode current conducting member attached to the cathode;
   an anode current conducting member wherein the or each of the elongated member/s is conductively connected electrically with the anode current conducting member;
b) immersing said stent/s into the electrolytic solution;
c) supplying a voltage difference between the cathode current conducting member and the anode current conducting member;
d) removing the stent/s from the solution and rinsing with at least one rinsing solution;
e) optionally, repeating steps b), c), and d).

In one embodiment, the method further may include
f) removing the stents from the apparatus;
g) rinsing the stents;
h) immersing the stents in a passivation solution;
i) removing the stents from the passivation solution and rinsing;
j) placing the stents in a liquid and applying ultrasound energy to the liquid, or combinations thereof.

In one embodiment of the above method, the following proviso applies: with the proviso that said electrolytic solution may not at the same time comprise polyethylene glycol, dimethylsulfate, and ethanol.

In another embodiment of the above method, in act c) the voltage is supplied for a period in the range of about 20 to about 60 seconds while the stents are immersed in the electrolytic solution.

In a further embodiment of the above method, acts b), c), and d) are repeated three to five times.

In one embodiment of the above method, the passivation solution includes nitric acid.

An embodiment of an electrolytic solution is provided for electropolishing a metallic stents also for use in the method according to the invention described above, the electrolytic solution comprising dimethylsulfate.

In one embodiment of the electrolytic solution, the following proviso applies: with the proviso that said electrolytic solution may not at the same time comprise polyethylene glycol, dimethylsulfate, and ethanol.

In one embodiment, the electrolytic solution may include from about 25 to about 60 weight percent dimethylsulfate, from about 30 to about 40 weight percent, from about 35 to about 39 weight percent, or about 37 weight percent.

In one embodiment, the electrolytic solution may include/comprise at least one additional component like at least one alcohol e.g. polyethylene glycol, or at least one alkyl alcohol like ethanol, and/or methanol.

In one embodiment, the electrolytic solution further comprises polyethylene glycol. The electrolytic solution may include from about 0.1 to 5 weight percent polyethylene glycol, from about 0.5 to about 2 weight percent polyethylene glycol, or about 0.5 to 1 weight percent polyethylene glycol. In a further embodiment, the polyethylene glycol is PEG 1000.
In one embodiment, the electrolytic solution further comprises at least one alkyl alcohol, preferably selected from ethanol or methanol or from a mixture of ethanol and methanol. The electrolytic solution may include from about 15 to about 50 weight percent ethanol, from about 20 to about 40 weight percent, from about 25 to about 35 weight percent, or about 28 weight percent. Preferably the balance of the electrolytic solution is made up of methanol.

These and other objects and features of the present disclosure will become more fully apparent from the following description and appended claims, or may be learned by the practice of the disclosure as set forth hereinafter.

### BRIEF DESCRIPTION OF THE DRAWINGS

To further clarify the above and other advantages and features of the present disclosure, a more particular description of the disclosure will be rendered by reference to specific embodiments thereof; use of which is illustrated in the appende drawings. The disclosure will be described and explained with additional specificity and detail through the use of the accompanying drawing in which:

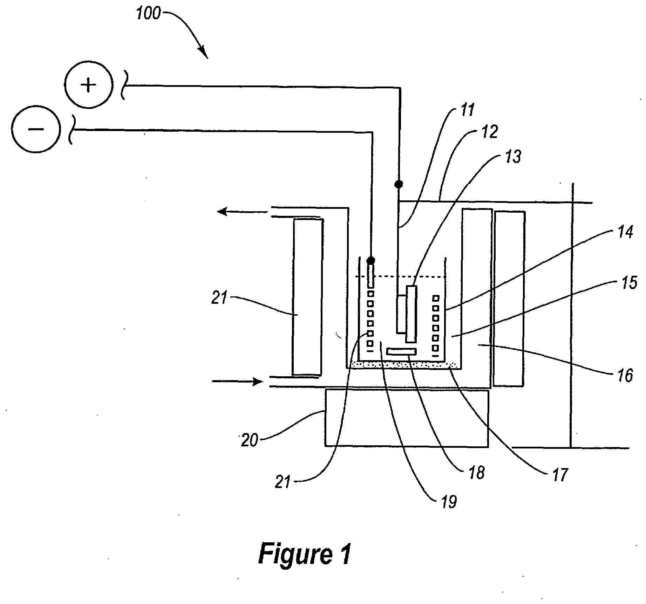

Figure 1 illustrates a view of an apparatus usable in the method or in use of the electrolytic solution according to the disclosure showing the stents immersed in the electrolytic solution and in close proximity to the cathode.

### DETAILED DESCRIPTION

The present disclosure is directed to a method and use of an electrolytic solution for electropolishing a metallic device or a plurality of metallic devices, in particular, metallic stents. The present disclosure may provide the advantage of providing a very easy way to polish the devices and/or providing a high degree of smoothness in the polished device and/or even allowing simultaneously electropolishing a plurality of devices. By providing a spiral cathode in an electropolishing container made from a material with low thermal conduction, the electrolyte solution may remain stable for an extended time.

In an apparatus in which the method and/or the electrolytic solution may be used, the apparatus provides a spiral cathode in an electropolishing container made from a material with low thermal conduction. Thus, the electrolyte solution may remain stable for an extended time in such an apparatus. The apparatus for example includes two concentric spiral cathodes with the stents, as anodes, placed therebetween, thereby providing additional cathode surface area. The pitch of the spiral can be varied in order to balance good agitation of the electrolytic solution in the polishing container to ensure continuous electrolyte concentration and composition and provision of a high surface area of the cathode.

The core of the invention described here is a novel use of an electrolytic solution comprising dimethylsulfate for improved electropolishing of metallic medical devices, in particular stents.

Referring to FIG. 1, there is shown an apparatus 100 which may be used in a method or with an electrolytic solution according to the disclosure. The apparatus may include at least one elongated member 11 in a downward orientation along a longitudinal axis. Each of the members 11 may accommodate an electrically conductive adapter which may be capable of being removably affixed to and/or in electrical contact with a metallic stent 13. The method according to the invention is herewith disclosed being exemplified in connection with such an apparatus. Still, the following proviso applies: with the proviso that said electrolytic solution/electropolishing solution 19 may not at the same time comprise polyethylene glycol, dimethylsulfate, and ethanol.

With the at least one elongated member 11 immersed in electropolishing solution 19 - the electrolytic solution comprising dimethylsulfate - contained in a container 14, a tubular continuous spiral cathode 21 may be in close proximity with the at least one elongated members 11. Each member 11 may be substantially equidistant from the facing surface of the cathode 21. This may provide a consistent field to each of the stents. Cathode 21 may be attached to a cathode collecting member. The at least one stent, as anodes, attached to elongated member(s) 11 may all be in electrical connection in series and/or in parallel with anode current conducting member. Cathode conducting member and/or anode conducting member may be connected to an electromotive force (EMF)-providing DC source, such as a battery, from which current and/or voltage may be controlled by an appropriate controller. The entire system may be placed into a polishing container 14 made from material with a low thermal conduction. Suitable materials may include glass, ceramics, plastic, or other materials. The polishing container 14 may contain the electropolishing solution 19 - the electrolytic solution comprising dimethylsulfate - and may be placed in a double walled reaction container 16. The polishing container 14 may be cooled with a cooling solution 15 like e.g. Ethanol. Using a polishing container 14 made from material with a low thermal conduction water may reduce condensation at the wall portions of the polishing container exposed to air. A dilution of the polishing solution - the electrolytic solution comprising dimethylsulfate - by condensing water out of the air may be reduced potentially resulting in an electropolishing solution 19 - the electrolytic solution comprising dimethylsulfate - that may be stable and/or reliable and/or may provide constant - even improved - polishing results. To facilitate agitation of the electropolishing solution - the electrolytic solution comprising dimethylsulfate - during the electropolishing process, a stirrer 18, such as a magnetic stirrer may be placed into the polishing container 14.

Typical coronary and/or endovascular stents may vary in a range from about 7 to up to about 200 millimeters in length with a diameter in a range of about 1 to about 12 millimeters. However, stents of larger or smaller size may be suitably accommodated for the method according to the invention in such an apparatus.

In order to accomplish the electropolishing process/the method according to the invention, the stents, which may all be identical in length, diameter, design, or combinations there of, may be placed on one or more of the adaptors. The mounted stents may be immersed into the electropolishing solution - the electrolytic solution according to the invention comprising dimethylsulfate. The temperature of the electropolishing solution - the electrolytic solution comprising dimethylsulfate - may be between about -20°C and about 0°C, between about -20°C and about -10°C, or about -15°C. A voltage is supplied to the stents, as anodes, and the cathode to electropolish the stents to the desired smoothness. Useful voltage may be in the range of about 7 to about 40 volts, between about 10 and about 30 volts, or between about 10 and about 20 volts. Voltage may be applied in about 20 to about 60 second intervals, in some embodiments in about 30 second intervals. However, voltages outside of these ranges may also be useful, depending upon the number of stents, electrolyte, and/or other design and/or process parameters.

It may be desirable for the electropolishing process/method according to the invention to be performed in stages. After one immersion in the electropolishing solution - the electrolytic solution comprising dimethylsulfate -, typically lasting from about 20 to about 60 seconds, the stents may be removed from the solution and washed, typically with alcohol, water, nitric acid, or combinations thereof. Then, the electropolishing may be repeated several times with each step followed by a rinse of the stents. Typically a suitable polishing process/method according to the invention will include about three to about five iterations of the electropolishing step. But more or fewer iterations may be suitable, depending upon the stents, electrolyte, voltage, other process variations, or combinations thereof. Once the desired electropolishing is completed, the stents may be removed from the electropolishing solution - the electrolytic solution comprising dimethylsulfate - and from the electropolishing apparatus, rinsed, and contacted with a passivation solution to remove residual electropolishing solution. The stents are typically again rinsed and placed in a bath to which ultrasound energy may be applied to complete the rinsing. A final rinse step may involve exposure for about 10 minutes in an ultrasound bath at approximately room temperature. For the electrolytic solution comprising dimethylsulfate, the following proviso applies: with the proviso that said electrolytic solution may not at the same time comprise polyethylene glycol, dimethylsulfate, and ethanol.

In one embodiment, the electrolytic solution may include from about 25 to about 60 weight percent dimethylsulfate, from about 30 to about 40 weight percent, from about 35 to about 39 weight percent, or about 37 weight percent.

In one embodiment of the electrolytic (electropolishing) solution the electrolytic solution may further include/comprise at least one additional component like at least one alcohol e.g. polyethylene glycol, or at least one alkyl alcohols like ethanol, and methanol.

In one embodiment, the electrolytic solution further comprises polyethylene glycol.

In one embodiment, the polyethylene glycol is PEG 1000.

In a further embodiment, the electrolytic solution may include from about 0.1 to about 5 weight percent polyethylene glycol, from about 0.5 to about 2 weight percent polyethylene glycol, or about 0.5 to 1 weight percent polyethylene glycol.

In one embodiment, the electrolytic solution further comprises at least one alkyl alcohol, preferably selected from ethanol or methanol or from a mixture of ethanol and methanol. The electrolytic solution may include from about 15 to about 50 weight percent ethanol, from about 20 to about 40 weight percent, from about 25 to about 35 weight percent, from about 25 to about 30 weight percent or about 28 weight percent. Preferably the balance of the electrolytic solution is made up of methanol.

The following example is presented for the purpose of illustration and is not intended to limit the disclosure in any way.

### EXAMPLE

Four dry identical stents made from high strength medical alloy may be removably affixed to the adapters of four elongated members. While agitating the electropolishing solution comprising dimethylsulfate, in this example which is not covered by the invention, composed of: polyethylene glycol (PEG 1000) : dimethylsulfate : ethanol: methanol in a weight electropolishing solution according to the invention comprising dimethylsulfate (in this ratio of about 1 : 59 : 44: 54, i.e. 0.6 weight percent polyethylene glycol (PEG 1000), 37.3 weight percent dimethylsulfate, 27.9 weight percent ethanol and 34.2 weight percent methanol) the stents are lowered on the apparatus into the electropolishing solution. The positive lead from the electrical source is attached to the apparatus and the magnetic stirrer in the electropolishing container is turned on to facilitate agitation of the electropolishing solution. When the cycle time has elapsed (depending on the size and type of stent), the stents are removed from the electropolishing solution and submerged in a container of ethanol. Each stent is moved while submerged. The stents are then re-immersed in the electropolishing solution for another polishing cycle. The polishing cycle is repeated for four polishing cycles. The stents are removed from the adapters and placed into a purified water rinse for about 30 seconds. The stents are then removed and placed in nitric acid passivation rinse bath for 30 minutes. The stents are removed from the bath and placed in a purified water ultrasonic bath for about 10 minutes. The stents are then removed from the bath, rinsed with alcohol and are dried with compressed air. The stents achieved a very high degree of smoothness.

## Claims

1. A method of electropolishing metallic stents comprising:
a) affixing a stent on each of one or more of electrically conductive adaptors of an apparatus, said apparatus comprising:
at least one elongated member having a longitudinal axis, each of said at least one member comprising an electrically conductive adaptor capable of being removably affixed to and in electrical contact with a metallic stent;
an electrolytic solution comprising dimethylsulfate;
a continuous cathode configured to be located in close proximity to each of said at least one elongated member when said a least one elongated member and cathode are immersed in said electrolytic solution;
a cathode current conducting member attached to said cathode;
an anode current conducting member wherein the said member or each of said elongated members is conductively connected electrically with said anode current conducting member;
b) immersing said at least one stent in said electrolytic solution;
c) supplying a voltage difference between said cathode current conducting member and said anode current conducting member;
d) removing said at least one stent from said solution and rinsing with alcohol; and
e) optionally, repeating acts b), c), and d);
with the proviso that said electrolytic solution may not at the same time comprise polyethylene glycol, dimethylsulfate, and ethanol.

2. The method according to claim 1, further comprising
f) removing said at least one stent from said apparatus;
g) rinsing said at least one stent;
h) immersing said at least one stent in a passivation solution;
i) removing said at least one stent from said passivation solution and rinsing said at least one stent; and
j) placing said at least one stent in a liquid and applying ultrasound energy to said liquid.

3. The method according to claim 1, wherein said steps b), c), and d) are repeated three to five times.

4. The method according to claim 2, wherein said passivation solution comprises nitric acid.

5. The method according to claim 2, wherein said ultrasound energy is applied to said liquid at room temperature.

6. Use of an electrolytic solution comprising dimethylsulfate for electropolishing of metallic medical devices, with the proviso that said electrolytic solution may not at the same time comprise polyethylene glycol, dimethylsulfate and ethanol.

7. Use of the electrolytic solution according to claim 6, comprising 25 to 60 weight percent dimethylsulfate.

8. Use of The electrolytic solution according to a claim 7, comprising 35 to 39 weight percent dimethylsulfate.

9. Use of the electrolytic solution according to claims 6 to 8, wherein the electrolytic solution further comprises at least one additional component, preferably at least one alcohol.

10. Use of the electrolytic solution according to claims 6 to 9, wherein the electrolytic solution further comprises polyethylene glycol, preferably comprises PEG 1000.

11. Use of the electrolytic solution according to claim 10, comprising 0.1 to 5 weight percent polyethylene glycol.

12. Use of the electrolytic solution according to claim 11, comprising 0.5 to 1 weight percent polyethylene glycol.

13. Use of the electrolytic solution according to any of claims 6 to 9, wherein the electrolytic solution further comprises at least one alkyl alcohol, preferably selected from ethanol or methanol or from a mixture of ethanol and methanol.

14. Use of the electrolytic solution according to claim 13, comprising 15 to 50 weight percent ethanol and a balance of methanol.

15. Use of the electrolytic solution according to a claim 14, comprising between 25 and 30 weight percent ethanol and a balance of methanol.

## Patentansprüche

1. Verfahren zum Elektropolieren metallischer Stents, mit folgenden Schritten:
a) Befestigen eines Stents an jedem von einem oder mehreren elektrisch leitenden Adaptern einer Vorrichtung, wobei die Vorrichtung enthält:
wenigstens ein längliches Element mit einer Längsachse, wobei jedes des wenigstens einen Elements einen elektrisch leitenden Adapter aufweist, der sich abnehmbar an einem metallischen Stent und in elektrischem Kontakt mit diesem befestigten lässt;
eine Elektrolytlösung, die Dimethylsulfat umfasst;
eine kontinuierliche Kathode, die angeordnet ist, um sich in unmittelbarer Nähe jedes des wenigstens einen länglichen Elements zu befinden, wenn das wenigstens eine längliche Element und die Kathode in der elektrolytischen Lösung eingetaucht sind;
ein kathodenstromführendes Element, das an der Kathode angebracht ist;
ein anodenstromführendes Element, wobei das Element oder jedes der länglichen Elemente elektrisch leitend mit dem anodenstromführenden Element verbunden ist;
b) Eintauchen des wenigstens einen Stents in die elektrolytische Lösung;
c) Bereitstellen einer Spannungsdifferenz zwischen dem kathodenstromführenden Element und dem anodenstromführenden Element;
d) Entfernen des wenigstens einen Stents aus der Lösung und Spülen mit Alkohol; und
e) optionales Wiederholen der Schritte b), c) und d);
mit der Bedingung, dass die Elektrolytlösung nicht gleichzeitig Polyethylenglykol, Dimethylsulfat und Ethanol aufweisen kann.

2. Verfahren nach Anspruch 1, ferner mit den Schritten:
f) Entfernen des wenigstens einen Stents aus der Vorrichtung;
g) Spülen des wenigstens einen Stents;
h) Eintauchen des wenigstens einen Stents in eine Passivierungslösung;
i) Entfernen des wenigstens einen Stents aus der Passivierungslösung und Spülen des wenigstens einen Stents; und
j) Platzieren des wenigstens einen Stents in einer Flüssigkeit und Anwenden von Ultraschallenergie auf die Flüssigkeit.

3. Verfahren nach Anspruch 1, wobei die Schritte b), c) und d) drei bis fünf Mal wiederholt werden.

4. Verfahren nach Anspruch 2, wobei die Passivierungslösung Salpetersäure umfasst.

5. Verfahren nach Anspruch 2, wobei die Ultraschallenergie auf die Flüssigkeit bei Raumtemperatur angewendet wird.

6. Verwendung einer Elektrolytlösung, die Dimethylsulfat zum Elektropolieren metallischer medizinischer Vorrichtungen aufweist, mit der Bedingung, dass die Elektrolytlösung nicht gleichzeitig Polyethylenglykol, Dimethylsulfat und Ethanol aufweisen kann.

7. Verwendung der elektrolytischen Lösung nach Anspruch 6, die 25 bis 60 Gewichtsprozent Dimethylsulfat enthält.

8. Verwendung der elektrolytischen Lösung nach einem Anspruch 7, die 35 bis 39 Gewichtsprozent Dimethylsulfat enthält.

9. Verwendung der elektrolytischen Lösung nach Anspruch 6 bis 8, wobei die elektrolytische Lösung ferner mindestens eine zusätzliche Komponente, vorzugsweise wenigstens einen Alkohol, enthält.

10. Verwendung der elektrolytischen Lösung nach Anspruch 6 bis 9, wobei die elektrolytische Lösung ferner Polyethylenglykol, vorzugsweise PEG 1000, enthält.

11. Verwendung der elektrolytischen Lösung nach Anspruch 10, die 0,1 bis 5 Gewichtsprozent Polyethylenglykol enthält.

12. Verwendung der elektrolytischen Lösung nach Anspruch 11, die 0,5 bis 1 Gewichtsprozent Polyethylenglykol enthält.

13. Verwendung der elektrolytischen Lösung nach einem beliebigen der Ansprüche 6 bis 9, wobei die elektrolytische Lösung ferner mindestens einen Alkylalkohol enthält, der vorzugsweise aus der aus Ethanol oder Methanol oder aus einem Gemisch von Ethanol und Methanol bestehenden Gruppe ausgewählt ist.

14. Verwendung der elektrolytischen Lösung nach Anspruch 13, die 15 bis 50 Gewichtsprozent Ethanol und den Rest Methanol enthält.

15. Verwendung der elektrolytischen Lösung nach einem Anspruch 14, die zwischen 25 und 30 Gewichtsprozent Ethanol und den Rest Methanol enthält.

## Revendications

1. Procédé de polissage électrolytique de stents métalliques consistant à :
a) fixer un stent sur chacun d'un ou de plusieurs adaptateurs qui conduisent électricité d'un appareil, ledit appareil comprenant :
au moins un élément allongé possédant un axe longitudinal, chacun desdits au moins un élément comprenant un adaptateur qui conduit l'électricité capable d'être fixé de manière détachable à un stent métallique et étant en contact électrique avec un stent métallique ;
une solution électrolytique comprenant du diméthylsulfate ;
une cathode continue configurée pour être située à proximité immédiate de chacun dudit au moins un élément allongé lorsque ledit au moins un membre allongé et la cathode sont immergés dans ladite solution électrolytique ;
un élément conduisant le courant cathodique attaché à ladite cathode ;
un élément conduisant le courant anodique dans lequel ledit élément ou chacun desdits éléments allongés est raccordé électriquement de manière conductible avec ledit élément conduisant le courant anodique ;
b) immerger ledit au moins un stent dans ladite solution électrolytique ;
c) fournir une différence de tension entre ledit élément conduisant le courant cathodique et ledit élément conduisant le courant anodique ;
d) éliminer ledit au moins un stent à partir de ladite solution et rincer avec de l'alcool ; et
e) éventuellement, répéter les étapes b), c) et d) ;
à condition que ladite solution électrolytique ne contienne pas au même moment du polyéthylène glycol, du diméthylsulfate et de l'éthanol.

2. Procédé selon la revendication 1, consistant en outre à
f) éliminer ledit au moins un stent à partir dudit appareil ;
g) rincer ledit au moins un stent ;
h) immerger ledit au moins stent dans une solution de passivation ;
i) éliminer ledit au moins un stent à partir de ladite solution de passivation et rincer ledit au moins stent ; et
j) placer ledit au moins stent dans un liquide et appliquer une énergie à ultrasons audit liquide.

3. Procédé selon la revendication 1, dans lequel lesdites étapes b), c) et d) sont répétées de trois à cinq fois.

4. Procédé selon la revendication 2, dans lequel ladite solution de passivation comprend de l'acide nitrique.

5. Procédé selon la revendication 2, dans lequel ladite énergie à ultrasons est appliquée audit liquide à température ambiante.

6. Utilisation d'une solution électrolytique comprenant du diméthylsulfate destinée au polissage électrolytique de dispositifs médicaux métalliques, à condition que ladite solution électrolytique ne contienne pas au même moment du polyéthylène glycol, du diméthylsulfate et de l'éthanol.

7. Utilisation de la solution électrolytique selon la revendication 6, comprenant de 25 à 60 pourcent en poids de diméthylsulfate.

8. Utilisation de la solution électrolytique selon la revendication 7, comprenant de 35 à 39 pourcent en poids de diméthylsulfate.

9. Utilisation de la solution électrolytique selon l'une quelconque des revendications 6 à 8, dans laquelle la solution électrolytique comprend en outre au moins un composant supplémentaire, de préférence au moins un alcool.

10. Utilisation de la solution électrolytique selon l'une quelconque des revendications 6 à 9, dans laquelle la solution électrolytique comprend en outre du polyéthylène glycol, de préférence comprend du PEG 1000.

11. Utilisation de la solution électrolytique selon la revendication 10, comprenant entre 0,1 et 5 pourcent en poids de polyéthylène glycol.

12. Utilisation de la solution électrolytique selon la revendication 11, comprenant entre 0,5 et 1 pourcent en poids de polyéthylène glycol.

13. Utilisation de la solution électrolytique selon l'une quelconque des revendications 6 à 9, dans laquelle la solution électrolytique comprend en outre au moins un alkylalcool, de préférence choisie parmi l'éthanol ou le méthanol ou à partir d'un mélange d'éthanol et de méthanol.

14. Utilisation de la solution électrolytique selon la revendication 13, comprenant entre 15 et 50 pourcent en poids d'éthanol et un reliquat de méthanol.

15. Utilisation de la solution électrolytique selon la revendication 14, comprenant entre 25 et 30 pourcent en poids d'éthanol et un reliquat de méthanol.
